# FASCICULE DE BREVET EUROPEEN

(11) **EP 0 680 745 B1**
(45) Date de publication et mention de la délivrance du brevet: **06.11.2002**
(21) Numéro de dépôt: 95400859.5
(22) Date de dépôt: 14.04.1995
(51) Int. Cl.: A61K 7/06, A61K 31/135, A61K 31/44, A61K 33/04, A61K 35/04, A61K 31/19, A61K 31/155, A61K 31/085, A61K 31/165

(54) **Utilisation de composés antifongiques et de composés antibactériens halogénés pour diminuer la chute des cheveux**
Verwendung von antimykotischen Mitteln und von halogenierten antibakteriellen Mitteln zur Verringerung des Haarausfalls
Use of antifungals and or halogenated antibacterial agents for diminishing hair loss

(30) Priorité: 05.05.1994 FR 9405541
(43) Date de publication de la demande: 08.11.1995
(60) Demande divisionnaire: 02009426.4
(73) Titulaire: L'OREAL, 75008 Paris (FR)
(72) Inventeur: Saint-Leger, Didier, F-92400 Courbevoie (FR)
(74) Mandataire: Tezier Herman, Béatrice

(56) Documents cités:
- WO-A-93/07847
- FR-A- 2 618 068
- FR-A- 2 685 867
- FR-A- 2 694 694
- GB-A- 2 197 194
- REYNOLDS, J.E.F. (ED.), 'MARTINDALE: THE EXTRA PHARMACOPOEIA' (ELECTRONIC VERSION). MICROMEDEX, INC., DENVER., XP002024644
- REYNOLDS, J.E.F. (ED.), 'MARTINDALE: THE EXTRA PHARMACOPOEIA' (ELECTRONIC VERSION). MICROMEDEX, INC., DENVER., XP002024645
- REYNOLDS, J.E.F. (ED.), 'MARTINDALE: THE EXTRA PHARMACOPOEIA' (ELECTRONIC VERSION). MICROMEDEX, INC., DENVER., XP002024646
- REYNOLDS, J.E.F. (ED.), 'MARTINDALE: THE EXTRA PHARMACOPOEIA' (ELECTRONIC VERSION). MICROMEDEX, INC., DENVER., XP002024647
- REYNOLDS, J.E.F. (ED.), 'MARTINDALE: THE EXTRA PHARMACOPOEIA' (ELECTRONIC VERSION). MICROMEDEX, INC., DENVER., XP002024648
- V-I VADEMECUM INTERNACIONAL, MEDICOM S.A., MADRID, 1993., XP002024649
- Ch. Zviak et R.P.R. Dawber, Structure, fonction et propriétés physico-chimique du cheveu, dans Ch. Zviak, Science des traitements capillaires, Masson (p.12)

## Description

L'invention est relative à l'utilisation d'au moins un agent antifongique et d'au moins un agent antibactérien halogéné autre que ceux appartenant aux familles des macrolides et des pyranosides dans une composition cosmétique ou pour la préparation d'une composition dermatologique, lesdites compositions étant destinées au traitement de la chute des cheveux.
L'homme de l'art sait depuis longtemps que la chute naturelle des cheveux, chez l'homme, reflète globalement l'équilibre des follicules pileux entre les phases alternatives de pousse (phase anagène) et les phases de chute (phase télogène). Le rapport moyen du nombre de follicules en phase anagène à celui en phase télogène est de l'ordre de 9 (90/10). Le pourcentage de follicules en phase de repos (phase catagène) y apparaît comme étant très faible.

La chute ou perte naturelle des cheveux peut être estimée, en moyenne, à quelques cent cheveux par jour pour un état physiologique normal. Pour un état physiologique pathologique, elle peut atteindre plusieurs centaines par jour conduisant à l'alopécie.

D'autre part, il existe à la surface du cuir chevelu une flore microbiologique naturellement constituée de bactéries et de levures. Lorsque qu'un déséquilibre intervient dans la composition naturelle de cette flore, la chute des cheveux peut être augmentée.

Il est connu, par ailleurs, que certains facteurs tels qu'un déséquilibre hormonal, un stress physiologique, les carences alimentaires, peuvent accélérer le phénomène.

Afin de diminuer la chute des cheveux, il a été proposé dans le brevet FR 2 618 068 l'utilisation d'une composition contenant un antifongique éventuellement associé à un agent anti-inflammatoire et/ou à un agent antibiotique de la famille des macrolides ou des pyranosides. Cependant de telles compositions n'apportent pas entière satisfaction car bien que la diminution de la chute des cheveux soit plus marquée qu'avec l'utilisation d'un antifongique seul, l'emploi d'agents anti-inflammatoires n'est pas sans inconvénient. En effet, ces agents anti-inflammatoires ont tendance à provoquer des variations de la composition naturelle de la flore microbiologique, ce qui augmente les risques d'infection.
Les agents antibiotiques ne donnent pas non plus entière satisfaction car ils sont souvent instables dans les compositions cosmétiques ou dermatologiques. Ils entraînent de plus des phénomènes de résistance bactérienne, provoquant ainsi une moindre efficacité des compositions destinées à freiner la chute des cheveux.

L'emploi d'agents antibactériens n'entraîne pas ces phénomènes de résistance mais il apparaît que la plupart des agents antibactériens classiques inhibe l'action des agents antifongiques ce qui diminue également l'efficacité des compositions contenant ce type d'association.

La demanderesse a donc cherché à résoudre ce problème. Elle a découvert que des compositions destinées à freiner la chute des cheveux et contenant un agent antifongique sont plus efficaces lorsque celui-ci est associé à un agent antibactérien halogéné n'appartenant pas aux familles des macrolides et des pyranosides. De façon surprenante, il apparaît que ces agents antibactériens halogénés n'inhibent pas l'action des agents antifongiques comme cela est le cas avec les autres agents antibactériens non halogénés.

Dans l'art antérieur des compositions associant un agent antifongique associé à un agent antibactérien halogéné sont connues, particulièrement dans le traitement des désordres de la peau et du cuir chevelu (voir par exemple Reynolds, J.E.F. (ed), 'Martindale : the extra pharmacopoeia', (electronic version), Micromedex, Inc., Denver)

De plus, de tels agents antibactériens halogénés sont très stables lorsqu'ils sont incorporés dans les compositions de l'invention et ils n'entraînent aucune résistance bactérienne.

La présente invention concerne donc l'utilisation d'au moins un agent antifongique et d'au moins un agent antibactérien halogéné autre que ceux appartenant aux familles des macrolides et des pyranosides pour la préparation d'une composition cosmétique ou dermatologique destinée au traitement de la chute des cheveux.

De plus, on constate qu'après quelques semaines de traitement, l'apparence des cheveux est améliorée, ceux-ci étant en particulier plus brillants, plus légers et moins gras.

Selon l'invention, on entend par agent antifongique, toute substance capable d'inhiber ou d'empêcher la croissance des levures en particulier celles que l'on trouve à la surface de l'épiderme riche en glandes sébacées et notamment à la surface du cuir chevelu comme par exemple le *Pityrosporum ovale* et ses variétés (*Pityrosporum orbiculare et Malassezia furfur*)

Parmi les agents antifongiques employés selon l'invention on peut plus particulièrement citer la terbinafine, le zinc pyrithione, le sulfure de sélénium, les goudrons et leurs dérivés, l'acide undécylénique et ses sels, les dérivés d'hydroxypyridone tels que le CICLOPIROX : 6-cyclohexyl 1-hydroxy 4-méthyl 2-(1 H)-pyridone ou l'OCTOPIROX : 1 -hydroxy 4-méthyl 6-(2,4,4,-triméthylpentyl)-2-(1H)-pyridone.

Ces agents antifongiques sont de préférence présents dans les compositions conformes à l'invention à une concentration pouvant varier entre 0,01 et 5 % en poids environ par rapport au poids total de la composition. Encore plus préférentiellement, la concentration en agents antifongiques peut varier entre 0,1 et 2 % en poids par rapport au poids total de la composition.

Selon l'invention, on entend par agent antibactérien halogéné, toute substance comportant au moins un atome d'halogène et capable d'inhiber ou d'empêcher la croissance de la flore bactérienne présente à la surface de l'épiderme riche en glandes sébacées.

De préférence, les agents antibactériens halogénés utilisés dans la présente invention sont des dérivés benzéniques.

Parmi les agents antibactériens halogénés employés selon l'invention, on peut plus particulièrement citer les agents antibactériens chlorés tels que le Triclosan qui est le 5-chloro-2-(2,4-dichlorophénoxy)phénol, vendu sous la dénomination commerciale IRGASAN par la Société CIBA-GEIGY, la chlorhexidine et ses dérivés, le chloramphénicol et le 1-(4-chlorophenoxy)-1-(1-H-imidazolyl)-3,3-dimethyl-2-butanone, vendu sous la dénomination commerciale CLIMBAZOLE par la Société BAYER. Ces agents antibactériens halogénés sont de préférence présents dans les compositions conformes à l'invention à une concentration pouvant varier entre 0,01 et 10 % en poids environ par rapport au poids total de la composition. Encore plus préférentiellement, la concentration en agents antibactériens peut varier entre 0,1 et 2 % en poids par rapport au poids total de la composition.

Le rapport pondéral des agents antifongiques aux agents antibactériens halogénés peut varier dans de larges proportions. En particulier, ce rapport pondéral peut varier de préférence de 0,2 à 10.

Les compositions conformes à l'invention peuvent se présenter sous des formes diverses habituellement utilisées en cosmétique ou en dermatologie pour le traitement du cuir chevelu.

Elles peuvent se présenter plus particulièrement sous forme de lotions, de shampooings, de mousses, de crèmes, de gels, de sticks, de sprays, de baumes, de poudres, de savons solides ou liquides.

Le milieu physiologiquement acceptable est généralement constitué par de l'eau ou par un mélange d'eau et d'au moins un solvant organique acceptable d'un point de vue physiologique en vue d'une application topique. Parmi ces solvants, on peut mentionner l'acétone, les alcools inférieurs en C₁-C₄ tels que l'éthanol, l'alcool isopropylique, les alkylène-glycol tel que l'éthylène-glycol, le propylène-glycol, les éthers monométhylique, monoéthylique ou monobutylique de l'éthylène-glycol, les monoéthyléthers du propylène-glycol et du dipropylène-glycol, les esters d'alkyle en C₁-C₄ d'acide à chaîne courte et les éthers de polytétrahydrofuranne. Lorsqu'ils sont présents, ces solvants représentent de préférence de 1 à 80 % en poids du poids total de la composition.

Le milieu peut être épaissi à l'aide d'agents épaississants habituellement utilisés en cosmétique ou en pharmacie.

Parmi ces agents épaississants, on peut en particulier citer la cellulose et ses dérivés comme les éthers de cellulose, les hétérobiopolysaccharides tels que la gomme de xanthane, les scléroglucanes, les acides polyacryliques réticulés ou non.

Les agents épaississants sont présents de préférence dans des proportions variant entre 0,1 et 5 % en poids environ par rapport au poids total de la composition.

Suivant les diverses formes de présentation désirées des compositions, l'homme de métier saura choisir les composés et adjuvants nécessaires et habituellement utilisés pour la réalisation de ces compositions.

Parmi ces adjuvants, on peut notamment citer les agents conservateurs, les agents stabilisants, les agents régulateurs de pH, les agents modificateurs de pression osmotique, les agents émulsifiants, les filtres solaires, les agents antioxydants, les parfums, les colorants, les agents tensioactifs, anioniques, cationiques, non-ioniques, amphotères, zwittérioniques ou leurs mélanges, les polymères, etc...

Les compositions peuvent également contenir en plus de l'association particulière faisant l'objet de l'invention, des composés déjà connus pour freiner la chute des cheveux.

L'invention a également pour objet un procédé de traitement cosmétique des cheveux et/ou du cuir chevelu consistant à leur appliquer une composition telle que définie ci-dessus, en vue de diminuer leur chute.

Le mode d'application préféré consiste à appliquer 1 à 20 g de la composition, sur l'ensemble ou sur certaines parties du cuir chevelu, à une fréquence de une à deux applications par jour, pendant 1 à 7 jours par semaine et ceci pendant une durée de 1 à 6 mois.

Les exemples suivants sont destinés à illustrer l'invention.

### Exemple 1 : on prépare un shampooing de composition suivante :

- Lauryl éther sulfate de sodium à 2,2 moles d'oxyde d'éthylène 12 g
- Monoisopropanolamide de coprah 3,5 g
- Hydroxypropylcellulose quaternisée par la triéthanolamine 0,4 g
- 1-hydroxy 4-méthyl 6-(2,4,4,-triméthylpentyl)-2-(1 H)-pyridone, vendu sous la dénomination commerciale OCTOPIROX par la société HOECHST 0,3 g
- Triclosan vendu sous la dénomination commerciale IRGASAN par la Société CIBA-GEIGY 0,25 g
- Conservateur 0,3 g
- Parfum 0,4 g
- Eau qsp 100 g

Ce shampooing, utilisé régulièrement à raison de 2 à 3 fois par semaine permet de diminuer la chute des cheveux tout en améliorant l'aspect général de la chevelure.

### Exemple 2 : on prépare une lotion pour le cuir chevelu de composition suivante :

- Alcool éthylique 38 g
- Huile de ricin 0,2 g
- Gluconate de chlorhexidine vendu par la société I.C.I. 0,4 g
- Acide undécylénique 0,25 g
- Parfum 0,3 g
- Colorant 0,05 g
- Eau qsp 100 g

Cette lotion, appliquée quotidiennement sur le cuir chevelu et les cheveux, sans rinçage, permet de diminuer la chute des cheveux tout en améliorant l'aspect général de la chevelure.

### Exemple 3 : on prépare une mousse pour le cuir chevelu de composition suivante :

- Polymère de chlorure de diallyldiméthyl ammonium, vendu sous la dénomination MERQUAT 100 par la société MERCK 1,5 g
- Copolymère d'hydroxyéthylcellulose et de chlorure de diallyldiméthyl ammonium vendu sous la dénomination CELQUAT LOR par la société NATIONAL STARCH 0,3 g
- Sel d'ammonium quaternaire vendu sous la dénomination ARQUAD 16-25W par la société AKZO 0,3 g
- Gomme de silicone vendue sous la dénomination QC F2 1671 par la société DOW CORNING 0,1 g
- Propylène glycol 5 g
- Phénoxyéthanol 0,4 g
- 1-hydroxy 4-méthyl 6-(2,4,4,-triméthylpentyl)-2-(1H)-pyridone, vendu sous la dénomination commerciale OCTOPIROX par la société HOECHST 0,2 g
- Chloramphénicol 0,3 g
- Eau qsp 100 g
- Propulseur : hydrocarbures (mélange isobutane /butane / propane dans des proportions 55 / 23 / 22).

Après application régulière de cette mousse, on observe une diminution de la chute des cheveux et une amélioration de l'aspect général de la chevelure.

### Exemple 4 : on prépare un spray pour le cuir chevelu de composition suivante :

- Hydrocarbure isoparaffinique (isobutane vendu par la société HULS) 0,5 g
- Polydiméthylsiloxane vendu sous la dénomination DC 200 FLUID par la société DOW CORNING 1,2 g
- Polyamino siloxane vendu sous la dénomination DC 929 EMULSION par la société DOW CORNING 0,4 g
- Ethanol 15 g
- Polymère d'acide acrylique réticulé vendu sous la dénomination CARBOPOL 980 par la société GOODRICH 0,1 g
- Triclosan vendu sous la dénomination commerciale IRGASAN par la Société CIBA-GEIGY 0,35 g
- 1-hydroxy 4-méthyl 6-(2,4,4,-triméthylpentyl)-2-(1H)-pyridone, vendu sous la dénomination commerciale OCTOPIROX par la société HOECHST 0,1 g
- Acide undécylénique 0,15 g
- Triéthanolamine qs pH 7
- Eau qsp 100 g

Cette composition est conditionnée en flacon-pompe.
Après application régulière de ce spray, on observe une diminution de la chute des cheveux et une amélioration de l'aspect général de la chevelure.

### Exemple 5 : on prépare une lotion antichute de composition suivante :

- 1-(4-chlorophenoxy)-1-(1-H-imidazolyl)-3,3-dimethyl-2-butanone, vendu sous la dénomination commerciale CLIMBAZOLE par la Société BAYER 0,1 g
- Acide undécylénique 0,15 g
- Ethanol 38 g
- parfum qs
- colorant qs
- eau qsp 100 g

Cette lotion, appliquée quotidiennement sur les cheveux, sans rinçage, permet de diminuer la chute des cheveux tout en améliorant l'aspect général de la chevelure.

### Exemple 6 : Test d'efficacité

Afin de mettre en évidence l'efficacité des compositions de l'invention, la lotion suivante a été préparée :
- 1-hydroxy 4-méthyl 6-(2,4,4,-triméthylpentyl)-2-(1H)-pyridone, vendu sous la dénomination commerciale OCTOPIROX par la société HOECHST 0,25 g
- Triclosan vendu sous la dénomination commerciale IRGASAN par la Société CIBA-GEIGY 0,3 g
- Ethanol 45 g
- parfum qs
- eau qsp 100 g

Cette lotion a été confiée à 20 personnes de sexe masculin qui présentaient une alopécie androgénique. Ils ont appliqué, cette composition sur leur cuir chevelu sans la rincer, à raison d'une fois par jour pendant 9 mois. Au cours du suivi de cette étude, un questionnaire standardisé a été remis à chaque personne périodiquement.
Les résultats observés figurent dans les tableaux ci-après :

### A ) ASPECT DES CHEVEUX

Ces personnes se sont prononcées quant au caractère gras, terne, collant ou normal de leur chevelure.

Les résultats figurent dans le tableau ci-après :

| **Temps en mois** | 0 | 0,5 | 1 | 1,5 | 2 | 3 | 4 | 5 | 6 | 7 | 8 | 9 |
|---|---|---|---|---|---|---|---|---|---|---|---|---|
| **Chevelure grasse** | 19 | 5 | 3 | 4 | 2 | 3 | 1 | 3 | 2 | 2 | 2 | 3 |
| **Chevelure non grasse** | 1 | 15 | 17 | 16 | 18 | 17 | 19 | 17 | 18 | 18 | 18 | 17 |
| **Chevelure terne** | 7 | 6 | 6 | 3 | 2 | 1 | 1 | 1 | 1 | 1 | 2 | 1 |
| **Chevelure non terne** | 13 | 14 | 14 | 17 | 18 | 19 | 19 | 19 | 19 | 19 | 18 | 19 |
| **Chevelure collante** | 17 | 2 | 1 | 0 | 0 | 0 | 0 | 0 | 0 | 0 | 0 | 0 |
| **Chevelure non collante** | 3 | 18 | 19 | 20 | 20 | 20 | 20 | 20 | 20 | 20 | 20 | 20 |
| **Chevelure normale** | 1 | 13 | 16 | 16 | 18 | 17 | 16 | 16 | 17 | 17 | 18 | 18 |
| **Chevelure anormale** | 19 | 7 | 4 | 4 | 2 | 3 | 4 | 4 | 3 | 3 | 2 | 2 |

On constate que la très grosse majorité des personnes a observé une normalisation générale de l'état de sa chevelure dès les premières semaines de traitement notamment en ce qui concerne leur aspect gras, terne et collant.

### B) Evolution de la séborrhée

Ces personnes ont évalué les variations de leur séborrhée, qui pouvait être accrue, stable ou diminuée. Les résultats figurent dans le tableau ci-après :

| **Temps en mois** | 0,5 | 1 | 1,5 | 2 | 3 | 4 | 5 | 6 | 7 | 8 | 9 |
|---|---|---|---|---|---|---|---|---|---|---|---|
| **Séborrhée accrue** | 3 | 1 | 0 | 0 | 0 | 0 | 1 | 0 | 0 | 1 | 0 |
| **Séborrhée stable** | 15 | 11 | 14 | 15 | 14 | 14 | 12 | 13 | 12 | 11 | 10 |
| **Séborrhée diminuée** | 2 | 8 | 6 | 5 | 6 | 6 | 7 | 7 | 6 | 8 | 10 |

On constate qu'après une phase transitoire de séborrhée accrue chez une minorité de personnes, une normalisation de celle-ci s'est installée. Aucune séborrhée réactionnelle n'a été décelée.

### C ) Observation du prurit

Ces personnes ont rapporté la présence ou l'absence de prurit. Les résultats figurent dans le tableau ci-dessous :

| **Temps en mois** | 0 | 0,5 | 1 | 1,5 | 2 | 3 | 4 | 5 | 6 | 7 | 8 | 9 |
|---|---|---|---|---|---|---|---|---|---|---|---|---|
| **Prurit** | 16 | 12 | 0 | 0 | 0 | 0 | 1 | 0 | 1 | 0 | 1 | 0 |
| **Absence de prurit** | 4 | 8 | 20 | 20 | 20 | 20 | 19 | 20 | 19 | 20 | 19 | 20 |

On constate qu'une très nette amélioration s'est installée dès les premières semaines de traitement.

### D ) Observation de la perte des cheveux

La perte des cheveux a été estimée par ces personnes. A chaque consultation, des enveloppes renfermant des cheveux d'une couleur proche de la leur et en nombres différents allant de 1 dizaine à 15 dizaines de cheveux leur ont été montrées. Chaque personne désignait alors l'enveloppe qui correspondait le mieux à l'estimation de la perte de ses cheveux lors du shampooing.
Les résultats sont présentés selon que la perte des cheveux augmente, reste stable ou diminue au long de l'application.
Ces résultats sont présentés dans le tableau ci-après :

| **Temps en mois** | 0,5 | 1 | 1,5 | 2 | 3 | 4 | 5 | 6 | 7 | 8 | 9 |
|---|---|---|---|---|---|---|---|---|---|---|---|
| **Perte de cheveux accrue** | 9 | 3 | 1 | 0 | 0 | 0 | 0 | 0 | 0 | 0 | 0 |
| **Perte de cheveux stable** | 8 | 13 | 12 | 13 | 12 | 10 | 6 | 7 | 7 | 7 | 8 |
| **Perte de cheveux diminuée** | 3 | 4 | 7 | 7 | 8 | 10 | 14 | 13 | 13 | 13 | 12 |

On constate, pour la majorité des personnes, une diminution significative de la chute des cheveux après quelques semaines d'application de la composition. Cette diminution a tendance à se stabiliser après 5 à 6 mois d 'application.

## Revendications

1. Utilisation non thérapeutique d'une composition cosmétique comprenant au moins un agent antifongique et au moins un agent antibactérien halogéné autre que ceux appartenant aux familles des macrolides et des pyranosides pour le traitement de la chute des cheveux.

2. Utilisation d'au moins un agent antifongique et d'au moins un agent antibactérien halogéné autre que ceux appartenant aux familles des macrolides et des pyranosides pour la préparation d'une composition dermatologique, ladite composition étant destinée au traitement de la chute des cheveux.

3. Utilisation selon l'une quelconque des revendications 1 ou 2, **caractérisée par le fait que** les agents antifongiques sont choisis dans le groupe formé par les substances capables d'inhiber ou d'empêcher la croissance des levures présentes à la surface de l'épiderme riche en glandes sébacées.

4. Utilisation selon l'une quelconque des revendications 1 à 3, **caractérisée par le fait que** les agents antifongiques sont choisis parmi les composés suivants : la terbinafine, le zinc pyrithione, le sulfure de sélénium, les goudrons et leurs dérivés, l'acide undécylénique et ses sels et les dérivés d'hydroxypyridone.

5. Utilisation selon la revendication 4, **caractérisée par le fait que** les dérivés d'hydroxypyridone sont choisis parmi la 6-cyclohexyl 1-hydroxy 4-méthyl 2-(1H)-pyridone et la 1-hydroxy 4-méthyl 6-(2,4,-triméthylpentyl) 2-(1H)-pyridone.

6. Utilisation selon l'une quelconque des revendications 1 à 5, **caractérisée par le fait que** les agents antibactériens halogénés sont choisis parmi les substances comportant au moins un atome d'halogène et capable d'inhiber ou d'empêcher la croissance de la flore bactérienne présente à la surface de l'épiderme riche en glandes sébacées.

7. Utilisation selon la revendication 6, **caractérisée par le fait que** les antibactériens halogénés sont des dérivés benzéniques.

8. Utilisation selon l'une quelconque des revendications 6 ou 7, **caractérisée par le fait que** les agents antibactériens halogénés sont des agents antibactériens chlorés.

9. Utilisation selon la revendication 8, **caractérisée par le fait que** les agents antibactériens chlorés sont choisis dans le groupe formé par les composés suivants : le 5-chloro-2-(2,4-dichlorophénoxy)phénol, la chlorhexidine et ses dérivés, le chloramphénicol et le 1-(4-chlorophenoxy)-1-(1 H-imidazolyl)-3,3-dimethyl-2-butanone.

10. Utilisation selon l'une quelconque des revendications 1 à 9, **caractérisée par le fait que** les agents antifongiques sont présents dans des proportions comprises entre 0,01 et 5 % en poids par rapport au poids total de la composition.

11. Utilisation selon l'une quelconque des revendications 1 à 10, **caractérisée par le fait que** les agents antifongiques sont présents dans des proportions comprises entre 0,1 et 2% en poids par rapport au poids total de la composition.

12. Utilisation selon l'une quelconque des revendications 1 à 11, **caractérisée par le fait que** les agents antibactériens halogénés sont présents dans des proportions comprises entre 0,01 et 10 % en poids par rapport au poids total de la composition.

13. Utilisation selon l'une quelconque des revendications 1 à 12, **caractérisée par le fait que** les agents antibactériens halogénés sont présents dans des proportions comprises entre 0,1 et 2 % en poids par rapport au poids total de la composition.

14. Utilisation selon l'une quelconque des revendications 1 à 13, **caractérisé par le fait que** le rapport pondéral de l'agent antifongique à l'agent antibactérien halogéné varie de 0,2 à 10.

15. Utilisation selon l'une quelconque des revendications 1 à 14, **caractérisée par le fait qu'**elle se présente sous forme de lotions, de shampooings, de mousses, de crèmes, de gels, de sticks, de sprays, de baumes, de poudres, de savons solides ou liquides.

16. Utilisation selon l'une quelconque des revendications 1 à 15, **caractérisée par le fait qu'**elle contient au moins un adjuvant choisi parmi les agents épaississants, les agents conservateurs, les agents stabilisants, les agents régulateurs de pH, les agents modificateurs de pression osmotique, les agents émulsifiants, les filtres solaires, les agents antioxydants, les parfums, les colorants, les polymères, les agents tensioactifs et tout autre adjuvant utilisé en application topique.

## Patentansprüche

1. Nicht-therapeutische Verwendung einer kosmetischen Zusammensetzung, die mindestens ein Antimykotikum und mindestens einen halogenierten antibakteriellen Wirkstoff enthält, der von den antibakteriellen Wirkstoffen verschieden ist, die zur Gruppe der Makrolide oder zur Gruppe der Pyranoside gehören, für die Behandlung des Haarausfalls.

2. Verwendung mindestens eines Antimykotikums und mindestens eines halogenierten antibakteriellen Wirkstoffs, der von den antibakteriellen Wirkstoffen verschieden ist, die zur Gruppe der Makrolide oder zur Gruppe der Pyranoside gehören, für die Herstellung einer dermatologischen Zusammensetzung, wobei diese Zusammensetzung für die Behandlung des Haarausfalls vorgesehen ist.

3. Verwendung nach einem der Ansprüche 1 und 2, **dadurch gekennzeichnet, daß** die Antimykotika unter den Substanzen ausgewählt werden, die imstande sind, das Wachstum von Hefen zu hemmen oder zu verhindern, die auf der Oberfläche der Epidermis vorhanden sind, die reich an Talgdrüsen ist.

4. Verwendung nach einem der Ansprüche 1 bis 3, **dadurch gekennzeichnet, daß** die Antimykotika unter den folgenden Verbindungen ausgewählt werden: Terbinafin, Zinkpyrithion, Selensulfid, den Teeren und ihren Derivaten, Undecylensäure und ihren Salzen und den Hydroxypyridon-Derivaten

5. Verwendung nach Anspruch 4, **dadurch gekennzeichnet, daß** die Hydroxypyridon-Derivate unter 6-Cyclohexyl-1-hydroxy-4-methyl-2-(1H)-pyridon und 1-Hydroxy-4-methyl-6-(2,4,4-trimethylpentyl)-2-(1H)-pyridon ausgewählt werden.

6. Verwendung nach einem der Ansprüche 1 bis 5, **dadurch gekennzeichnet, daß** die halogenierten antibakteriellen Wirkstoffe unter den Substanzen ausgewählt werden, die mindestens ein Halogenatom enthalten und imstande sind, das Wachstum der Bakterienflora, die auf der Oberfläche der Epidermis vorhanden ist, die reich an Talgdrüsen ist, zu hemmen oder zu verhindern.

7. Verwendung nach Anspruch 6, **dadurch gekennzeichnet, daß** die halogenierten antibakteriellen Wirkstoffe Benzolderivate sind.

8. Verwendung nach einem der Ansprüche 6 und 7, **dadurch gekennzeichnet, daß** die halogenierten antibakteriellen Wirkstoffe chlorhaltige antibakterielle Wirkstoffe sind.

9. Verwendung nach Anspruch 8, **dadurch gekennzeichnet, daß** die chlorhaltigen antibakteriellen Wirkstoffe unter den folgenden Verbindungen ausgewählt werden: 5-Chlor-2-(2,4-dichlorphenoxy)phenol, Chlorhexidin und seinen Derivaten, Chloramphenicol und 1-(4-Chlorphenoxy)-1-(1-H-imidazolyl)-3,3-dimethyl-2-butanon.

10. Verwendung nach einem der Ansprüche 1 bis 9, **dadurch gekennzeichnet, daß** die Antimykotika in Mengenanteilen enthalten sind, die im Bereich von 0,01 bis 5 Gew.-%, bezogen auf das Gesamtgewicht der Zusammensetzung, liegen.

11. Verwendung nach einem der Ansprüche 1 bis 10, **dadurch gekennzeichnet, daß** die Antimykotika in Mengenanteilen enthalten sind, die im Bereich von 0,1 bis 2 Gew.-%, bezogen auf das Gesamtgewicht der Zusammensetzung, liegen.

12. Verwendung nach einem der Ansprüche 1 bis 11, **dadurch gekennzeichnet, daß** die halogenierten antibakteriellen Wirkstoffe in Mengenanteilen enthalten sind, die im Bereich von 0,01 bis 10 Gew.-%, bezogen auf das Gesamtgewicht der Zusammensetzung, liegen.

13. Verwendung nach einem der Ansprüche 1 bis 12, **dadurch gekennzeichnet, daß** die halogenierten antibakteriellen Wirkstoffe in Mengenanteilen enthalten sind, die im Bereich von 0,1 bis 2 Gew.-%, bezogen auf das Gesamtgewicht der Zusammensetzung, liegen.

14. Verwendung nach einem der Ansprüche 1 bis 13, **dadurch gekennzeichnet, daß** das Gewichtsverhältnis des Antimykotikums zu dem halogenierten antibakteriellen Wirkstoff im Bereich von 0,2 bis 10 variiert.

15. Verwendung nach einem der Ansprüche 1 bis 14, **dadurch gekennzeichnet, daß** sie in Form von Lotionen, Haarwaschmitteln, Schäumen, Cremes, Gels, Sticks, Sprays, Balsams, Pudern, festen oder flüssigen Seifen vorliegt.

16. Verwendung nach einem der Ansprüche 1 bis 15, **dadurch gekennzeichnet, daß** sie mindestens einen Hilfsstoff enthält, der ausgewählt ist unter den Verdickungsmitteln, den Konservierungsmitteln, den Stabilisierungsmitteln, den den pH-Wert regulierenden Mitteln, den Mitteln zur Änderung des osmotischen Drucks, den Emulgatoren, den Sonnenschutzfiltern, den Antioxidantien, den Parfüms, den Farbmitteln, den Polymeren, den grenzflächenaktiven Stoffen und allen sonstigen bei der topischen Anwendung verwendeten Hilfsstoffen.

## Claims

1. Non-therapeutic use of a cosmetic composition comprising at least one antifungal agent and at least one halogenated antibacterial agent, other than those belonging to the macrolide and pyranoside families, for the treatment of hair loss.

2. Use of at least one antifungal agent and at least one halogenated antibacterial agent, other than those belonging to the macrolide and pyranoside families, for the preparation of a dermatological composition, the said composition being intended for the treatment of hair loss.

3. Use according to either of Claims 1 and 2, **characterized in that** the antifungal agents are chosen from the group formed by substances capable of inhibiting or preventing the growth of the yeasts present at the surface of the epidermis which is rich in sebaceous glands.

4. Use according to any one of Claims 1 to 3, **characterized in that** the antifungal agents are chosen from the following compounds: terbinafine, zinc pyrithione, selenium sulphide, tars and derivatives thereof, undecylenic acid and salts thereof and hydroxypyridone derivatives.

5. Use according to Claim 4, **characterized in that** the hydroxypyridone derivatives are chosen from 6-cyclohexyl-1-hydroxy-4-methyl-2-(1H)-pyridone and 1-hydroxy-4-methyl-6-(2,4,-trimethylpentyl)-2-(1H)-pyridone.

6. Use according to any one of Claims 1 to 5, **characterized in that** the halogenated antibacterial agents are chosen from substances containing at least one halogen atom and capable of inhibiting or preventing the growth of the bacterial flora present at the surface of the epidermis which is rich in sebaceous glands.

7. Use according to Claim 6, **characterized in that** the halogenated antibacterial agents are benzene derivatives.

8. Use according to either of Claims 6 and 7, **characterized in that** the halogenated antibacterial agents are chlorinated antibacterial agents.

9. Use according to Claim 8, **characterized in that** the chlorinated antibacterial agents are chosen from the group formed by the following compounds: 5-chloro-2-(2,4-dichlorophenoxy)phenol, chlorhexidine and derivatives thereof, chloramphenicol and 1-(4-chlorophenoxy)-1-(1H-imidazolyl)-3,3-dimethyl-2-butanone.

10. Use according to any one of Claims 1 to 9, **characterized in that** the antifungal agents are present in proportions between 0.01 and 5% by weight relative to the total weight of the composition.

11. Use according to any one of Claims 1 to 10, **characterized in that** the antifungal agents are present in proportions between 0.1 and 2% by weight relative to the total weight of the composition.

12. Use according to any one of Claims 1 to 11, **characterized in that** the halogenated antibacterial agents are present in proportions between 0.01 and 10% by weight relative to the total weight of the composition.

13. Use according to any one of Claims 1 to 12, **characterized in that** the halogenated antibacterial agents are present in proportions between 0.1 and 2% by weight relative to the total weight of the composition.

14. Use according to any one of Claims 1 to 13, **characterized in that** the weight ratio of the antifungal agent to the halogenated antibacterial agent ranges from 0.2 to 10.

15. Use according to any one of Claims 1 to 14, **characterized in that** the composition is in the form of lotions, shampoos, foams, creams, gels, sticks, sprays, balms, powders or solid or liquid soaps.

16. Use according to any one of Claims 1 to 15, **characterized in that** the composition contains at least one adjuvant chosen from thickening agents, preserving agents, stabilizing agents, pH regulators, osmotic pressure modifiers, emulsifying agents, sunscreen agents, antioxidants, fragrances, dyes, polymers, surface-active agents and any other adjuvant used in topical application.
